(19) **Europäisches Patentamt**
**European Patent Office**
**Office européen des brevets**

(11) **EP 2 168 607 B1**

(12) **FASCICULE DE BREVET EUROPEEN**

(45) Date de publication et mention
de la délivrance du brevet:
**17.08.2011 Bulletin 2011/33**

(51) Int Cl.:
*A61L 15/22* *(2006.01)* *A61L 24/04* *(2006.01)*
*A61L 26/00* *(2006.01)*

(21) Numéro de dépôt: **09170911.3**

(22) Date de dépôt: **22.09.2009**

(54) **Interface chirurgicale pour plaie, sans support**

Trägerfreie chirurgische Schnittstelle für Wunde

Self-supported surgical interface for wound

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **24.09.2008 FR 0805247**

(43) Date de publication de la demande:
**31.03.2010 Bulletin 2010/13**

(73) Titulaire: **PLASTO**
**21300 Chenove (FR)**

(72) Inventeurs:
• **Staeger épouse Williams, Maud**
**21000, Dijon (FR)**

• **Guillemet, Alain**
**21121, Fontaine-Les-Dijon (FR)**

(74) Mandataire: **Bertrand, Didier et al**
**S.A. Fedit-Loriot**
**38 Avenue Hoche**
**75008 Paris (FR)**

(56) Documents cités:
**WO-A-01/70285      WO-A-99/27014**
**FR-A- 2 783 412      US-A- 5 492 943**
**US-A1- 2005 123 590**

**EP 2 168 607 B1**

**Description**

La présente invention concerne une interface non adhérente destinée à être appliquée directement au contact d'une plaie.

Les interfaces connues sur le marché sont des interfaces comprenant une composition particulière disposée sur un support. Le document EP 1 143 895 décrit d'une part l'art antérieur général de l'invention auquel on renvoie, et d'autre part une interface non adhérente stérile, destinée à être appliquée directement au contact d'une plaie, comprenant un support en tissu flexible à mailles ouvertes dont les fils sont enrobés par un gel à matrice hydrophobe particulier, le gel enrobant les fils de manière à laisser les mailles essentiellement non obturées. Une telle interface donne satisfaction quant à son utilisation mais la technologie nécessaire pour obtenir un bon produit est délicate à mettre en oeuvre. En effet, il faut d'un côté que l'enrobage des fils soit absolument complet car il est indispensable d'éviter tout risque de contact direct entre une fibre nue du tissu de support et des bourgeons de cicatrisations de la plaie, et de l'autre côté, cet enrobage doit néanmoins être limité pour ne pas obstruer complètement les mailles et empêcher le passage des exsudats de la plaie vers l'extérieur de l'interface. Le document WO 99/27014 décrit une composition élastomérique utilisée comme pansement, la composition élastomérique est extrudée puis découpée avant stérilisation, les matrices polymériques ainsi produites sont directement appliquées sur la plaie. Cependant cette composition ne contient pas de polyoléfine et n'est pas trouée avant son utilisation gardant de ce fait la plaie en milieu humide. Le document US 2005/0123590 fait connaître une matrice hydrophobe éventuellement ternaire, dont les constituants peuvent être choisis dans des familles innombrables de matériaux aux propriétés très diverses et dans des proportions non définies. Cette matrice est systématiquement appliquée sur un matériau de substrat perméable définitif, c'est-à-dire que l'on ne retire pas lors de l'application sur la plaie. Le procédé de fabrication décrit au paragraphe 80, ou dans des variantes aux paragraphes 81 et 82 de ce document, montre que le substrat est initialement perméable et comprend des ouvertures substantielles, et que la composition lui est appliquée de manière à ne pas obstruer les ouvertures du substrat, ce qui pose des problèmes de fabrication.

Le but de l'invention est d'échapper à ce dilemme lié à la présence d'un support ou substrat définitif, notamment textile, et donc de proposer une interface autoportée, c'est à dire ne nécessitant pas de support ou d'armature.

Selon l'invention, on a découvert qu'il était possible d'obtenir une interface autoportée à partir d'une composition comprenant une matrice hydrophobe comportant A parties d'un élastomère tribloc, B parties d'un plastifiant, C parties d'une polyoléfine, et un hydrocolloïde dispersé dans une proportion comprise entre 0 et 30% du poids total de la matrice hydrophobe, avec les relations suivantes :

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

la composition d'interface étant disposée en couche mince sur un support provisoire, des trous traversants répartis étant réalisés dans l'interface

En pratique, l'interface est ôtée de son support provisoire pour être utilisée et elle présente une cohésion suffisante pour être manipulée et appliquée sur une plaie sans support ou armature, grâce à la composition particulière bien définie ci-dessus.

Selon l'invention, on réalise les trous dans l'interface seulement au moment où l'on dépose la composition sur un support protecteur provisoire ou même une fois la composition de l'interface déposée sur un support protecteur provisoire.

On peut ainsi obtenir selon l'invention un produit autoporté, neutre vis-à-vis de la plaie et de ses humeurs, et aéré.

De manière plus spécifique, lorsque la polyoléfine est le polyéthylène, le plastifiant une huile et lorsque la proportion d'hydrocolloïde est choisie entre 5 et 15%, l'invention concerne une interface autonome à partir d'une composition comprenant une matrice hydrophobe comportant A parties d'un élastomère tribloc, B parties d'huile de plastification, C parties de polyéthylène, et un hydrocolloïde dispersé dans une proportion comprise entre 5 et 15 % du poids total de la matrice hydrophobe, avec les relations suivantes :

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

la composition d'interface étant disposée en couche mince sur un support, des trous traversants répartis étant réalisés dans l'interface disposée sur le support, l'interface étant ôtée de son support pour être utilisée et présentant une cohésion suffisante pour être appliquée sur une plaie.

Mais l'invention n'est pas limitée à cet exemple spécifique et s'étend au contraire au cas où la polyoléfine n'est pas le polyéthylène et/ou le plastifiant n'est pas une huile et/ou la proportion d'hydrocolloïde n'est pas choisie entre 5 et 15%

L'invention concerne aussi le procédé de fabrication d'une interface chirurgicale pour plaie, caractérisé en ce que :

- 　on prépare une composition comprenant une matrice hydrophobe comportant A parties d'un élastomère tribloc, B parties d'un plastifiant, C parties d'une polyoléfine, et un hydrocolloïde dispersé dans une proportion comprise entre 0 et 30 % du poids total de la matrice hydrophobe, avec les relations suivantes :

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

- on enduit la composition d'interface en couche mince sur un support protecteur provisoire,
- et en ce qu'on réalise des trous traversants répartis dans cette interface.

Ces trous traversants sont réalisés simultanément à l'enduction sur le support protecteur provisoire ou après celle-ci.

L'invention concerne également une utilisation d'une interface chirurgicale pour plaie telle que définie ci-dessus, caractérisée en ce que l'interface est ôtée de son support protecteur provisoire pour être utilisée et présentent une cohésion suffisante pour être autoportée et donc manipulée et appliquée telle quelle sur une plaie sans support ni armature.

Par couche mince on entend typiquement une épaisseur de couche de l'interface comprise entre 50 $\mu$m et 2,5 mm , de préférence autour de 400 $\mu$m.

L'élastomère thermoplastique de synthèse, hydrophobe, de type tribloc, est avantageusement un élastomère de type SIS, formé par copolymérisation de blocs de type polystyrène (par exemple 15%) avec des blocs de type isoprène. L'élastomère a de préférence un poids moléculaire moyen ou élevé et son taux de diblocs est par exemple de 18 %. Un tel élastomère peut-être plastifié avec de l'huile (voir infra), mais un tel mélange s'avère donner, selon les taux d'huile, soit des produits gélatineux, mous sans aucune tenue, soit des produits élastiques sans aucun pouvoir collant ou happant.

Selon l'invention, c'est la polyoléfine qui apporte au mélange d'élastomère tribloc et d'huile la possibilité de rigidifier et rendre manipulable la composition finale, tout en présentant une inertie suffisante vis-à-vis de la peau, et en permettant le travail de la composition à chaud. Dans l'invention, la polyoléfine est préférentiellement du polyéthylène, apporté sous forme de granulés. Sa viscosité Brookfield se situe autour de 450 cps à 140°C et sa température de fusion comprise entre 92°C et 122°C. La quantité de polyéthylène apportée par rapport aux autres composants permet d'obtenir une bonne rigidité sans perte du caractère happant ni blanchiment du mélange. D'autres polyoléfines, tels que par exemple du polypropylène, pourront éventuellement être utilisés en complément ou en substitution du polyéthylène dans la composition pour assurer la fonction de rigidification de l'interface.

Le plastifiant de la composition est l'huile, notamment une huile minérale ou végétale compatible avec les autres éléments de la composition et tolérée par la peau. On utilise de préférence une huile de paraffine de faible viscosité à base de composés paraffiniques et naphténiques ou des mélanges d'huile de paraffine. D'autres plastifiants ou combinaisons de plastifiants, monomériques ou polymériques, compatibles avec tout ou partie des composants de la matrice, pourront également être utilisés dans l'invention.

La composition plastifiée est extensible et « élastique » et présente une certaine hystérésis avec un retour élastique d'au moins 3-5% pour un allongement de 20%.

Pour rendre plus anti-adhérent et pour maintenir au niveau de l'interface un environnement humide propice à la cicatrisation, on ajoute un hydrocolloïde en dispersion dans la matrice constituée des éléments ci-dessus définis. On pourra se référer au document précité EP 1 143 895 qui définit la nature et les conditions d'addition d'un hydrocolloïde convenant également à la présente invention. En pratique, on utilisera de préférence de la carboxyméthylcellulose de sodium (NaCMC) dans des proportions de 5% à 15% de la matrice hydrophobe. Ces proportions limitées sont importantes pour éviter l'effet de percolation en épaisseur qui entraînerait une absorption que l'invention ne recherche pas du tout, au contraire. D'autres particules absorbantes hydrophiles, en quantité réduite, pourront également être utilisées pour assurer le maintien de l'environnement humide à la surface de la plaie.

Naturellement, la composition peut comprendre également divers produits antioxydants ou stabilisants, charges, ainsi que des principes actifs qui ajouteront un effet particulier à la composition. Ces produits sont connus de l'homme du métier.

La fabrication de l'interface conforme à l'invention commence avec le malaxage à chaud et sans solvant selon la technique dite « hotmelt » (à une température d'environ 150°C) du polymère tribloc, du composant huileux, et du polyéthylène afin d'obtenir un mélange homogène, dans lequel on incorpore la CMC, à une température légèrement inférieure, par exemple, de 130°C-135°C. Le produit du mélange est ensuite déposé en enduction d'une épaisseur choisie sur un support. Le support peut être un support continu ou discontinu, il peut s'agir d'un support intermédiaire servant uniquement au process ou d'un support protecteur provisoire servant au transport et au stockage de l'interface jusqu'à son utilisation. Le support peut être de toute nature du moment qu'il présente une surface non-adhésive permettant la séparation facile de l'interface et du support. Le support protecteur provisoire est avantageusement un support souple, tel qu'une feuille (film, pellicule) siliconée, par exemple une feuille de polyester siliconé. On ajoute soit quasi simultanément soit lors d'une étape ultérieure une autre feuille siliconée au-dessus de l'interface de sorte que celle-ci est protégée sur ses deux faces jusqu'à application sur la plaie du patient. On pourra se référer pour une technique d'enduction entre deux supports à la figure page 153 de l'ouvrage « Coating and Laminating », Herbert Weiss, Converting Technology Machine, 1977.

Selon un aspect primordial de l'invention, le procédé prévoit la formation de trous dans l'interface enduite.

Selon un premier mode de réalisation, les trous sont pratiqués par une méthode de perforation (par exemple par poinçon et matrice) de l'enduction alors qu'elle est déjà déposée sur son support souple, et de préférence alors qu'elle est protégée par ses deux feuilles souples de protection.

Selon un second mode de réalisation, les trous sont formés dans l'enduction au moment même où celle-ci est déposée sur son support, par exemple par une machine commercialisée par Cavitec sous la référence Cavimel-TSM ou par Nordson sous la référence « system REA », ce procédé laissant automatiquement l'enduction sous forme d'une trame. Le principe est de déposer au moyen d'une buse le produit sur un cylindre tramé ou gravé qui ensuite transfère l'enduction tramée sur son support protecteur provisoire. Un tel procédé est par exemple connu par le document WO/ 011352.

Les trous présentent un diamètre moyen de 1 à 4 mm, par exemple sous forme de trous ronds de 3 mm avec un pas de 8 mm.

L'interface de l'invention a vocation à s'utiliser seule, une fois dégagée de son support protecteur ou de ses supports protecteur et déposée sur la plaie ; elle présente plusieurs caractéristiques favorables pour l'application :

- une certaine cohésion et une rigidité lui permettant d'être manipulée par le personnel soignant, puis d'être retirée entièrement sans laisser de résidus après utilisation
- une transparence permettant au personnel soignant de suivre l'évolution de la cicatrisation de la plaie sans retirer l'interface
- un pouvoir happant qui lui permet de rester seule en place sur la peau du patient, même si le patient change de position. Ce pouvoir happant est néanmoins exclusif d'une adhésivité notable au sens mesurable du terme, l'interface de l'invention étant précisément non collante et ne provoquant aucun arrachement de tissu humain ou de bourgeon de cicatrisation lors de son enlèvement.

Une fois l'interface conforme à l'invention placée sur une plaie, elle peut être associée à une couche supplémentaire, du côté opposé à la plaie, par exemple une compresse hydrophile (soit en gaze, soit en mousse de polyuréthanne telle que celle vendue sous les marques Rynel® et Corpura®, soit des non tissés absorbants tels que ceux commercialisés par la société Freudenberg) disposée au-dessus de l'interface.

Il est d'ailleurs possible d'associer cette couche supplémentaire à l'interface alors qu'elle est sur son support protecteur provisoire. Le complexe interface-couche supplémentaire est alors posé sur la plaie de manière unitaire, en plaçant l'interface du côté de la plaie, ce qui maintient la compresse à distance de la plaie et l'empêche ses fibres d'interférer avec la plaie ou la cicatrice.

Le tableau 1 ci-joint montre les données d'une première série d'expériences obtenues sur la base de 6 exemples. Dans chacun de ces exemples, on a procédé à la fabrication d'une composition dans les proportions indiquées sur le tableau à partir des produits suivants : Le polymère tribloc utilisé est un styrène-isoprène-styrène (SIS/SI) copolymère linéaire tricbloc/dibloc de marque Vector 4113A.

Le plastifiant est une huile blanche pharmaceutique sans goût ni odeur de marque Ondina 917 (Shell).

La polyéthylène est un homopolymère A-C 8 (Honeywell)

La NaCMC est une carboxyméthylcellulose de sodium purifiée grade 7H4XF.

Le stabilisant est un antioxydant phénolique de marque Irganox 1010 (Ciba).

On a malaxé d'abord la totalité de l'élastomère et 2/3 de l'huile à une température affichée de 175°C pendant 60 minutes puis on a ajouté la totalité du polyéthylène et le reste de l'huile. La chauffe du malaxeur a ensuite été arrêtée puis la NaCMC a été incorporée et mélangée pendant 30 minutes.

La composition a ensuite été déposée sur un support soit en enduction simple.

Des perforations rondes de diamètre 3 mm ont été faites à l'aide d'un outil rotatif de poinçonnage de circonférence 508 mm et de diamètre 161 mm.

On a ensuite évalué les propriétés des différents échantillons, en observant d'une part leur tenue (il faut que le produit ne soit ni trop mou, ni trop rigide ou cassant), leur couleur (il ne faut pas que le produit soit blanc et perde son caractère de transparence) et leur pouvoir happant, c'est-à-dire la faculté qu'ils ont de rester d'eux-mêmes sur la peau d'un patient, par exemple sur son bras retourné, sans tomber trop vite (on mesure le temps mis avant de tomber). Ces tests ont montré le bon comportement des échantillons 3 à 6 et ont confirmé l'intérêt des gammes retenues.

Pour confirmer ces données, on a ensuite testé un lot plus large d'échantillons de compositions comme rapporté dans le tableau 2 annexé. Les résultats montrent également que la conformité aux critères retenus dans le brevet a permis d'obtenir des échantillons présentant les bonnes caractéristiques fonctionnelles requises pour réaliser une interface sans support. Par ailleurs, on a effectué des mesures de viscosité Brookfield, des mesures de « rolling ball tack » (chemin en mm parcouru par une bille lancée sur un produit poisseux selon la norme ASTM D 3121) et des mesures de force en cN/cm lors d'un cycle d'allongement en traction à 20%. L'ensemble des mesures corrèle les observations faites selon lesquelles l'augmentation du taux de PE d'une part diminue le caractère happant de la composition et d'autre part rend le produit plus rigide, difficile à étirer et moins transparent, d'où le choix de la limite supérieure en PE selon l'invention.

Des tests classiques d'absorption réalisés sur des échantillons conformes à l'invention montrent une absorption inférieure à 1%, au contraire de proposition de l'art antérieur comme celle du document US 2005/0123590 qui recherche une absorption d'au moins 50% (paragraphe 0019).

**Tableau 1**

| Ex | 1 | 2 | 3 | 4 | 5 | 6 |
|---|---|---|---|---|---|---|
| A (SIS) | 100 | 100 | 100 | 100 | 100 | 100 |
| B (Huile) | 567 | 567 | 425 | 350 | 250 | 400 |
| C (PE) | 67 | 100 | 50 | 25 | 20 | 20 |
| CMC | | 76 | 57 | 47 | 77 | 51,5 |
| A+B+C | 734 | 767 | 575 | 475 | 370 | 520 |
| A/(A+B+C) | 13,6 | 13 | 17,4 | 21,1 | 27 | 19,2 |
| B/(A+B+C) | 77,2 | 73,9 | 73,9 | 73,7 | 67,6 | 76,9 |
| C/(A+B+C) | 9,1 | 13 | 8,7 | 5,3 | 5,4 | 3,8 |
| A/(A+B) | 15 | 15 | 19 | 22,2 | 28,6 | 20 |
| B/(A+B) | 85 | 85 | 81 | 77,8 | 71,4 | 80 |
| C/(A+C) | 40,1 | 50 | 33,3 | 20 | 16,7 | 16,7 |
| Module | - | 20,2 | 18,4 | 22,6 | 21,4 | - |
| Prop | cass. | cass. blanc gras | BT blanc | BT bon PH | cass bon PH | BT bon PH |
| Caract. fonct : | non OK | non OK | non OK | OK | non OK | OK |

BT = bonne tenue
PH = pouvoir happant
Cass. = cassant
Prop = propriétés
Caract. fonct. : caractéristiques fonctionnelles

**Tableau 2**

| Composition | Série HM-D2334 | | | | | | Série HM-D2327 | | | | |
|---|---|---|---|---|---|---|---|---|---|---|---|
| | A | B | C | D | E | F | A | B | C | D | E |
| A (SIS) | 100 | 100 | 100 | 100 | 100 | 100 | 100 | 100 | 100 | 100 | 100 |
| B (Huile) | 500 | 500 | 500 | 500 | 500 | 500 | 400 | 400 | 400 | 400 | 400 |
| C (PE) | 0 | 10 | 20 | 40 | 60 | 80 | 0 | 20 | 40 | 60 | 80 |
| Carboxy Methyl Cellulose | 59 | 60 | 61 | 63 | 65 | 67 | 49,5 | 51,5 | 53,5 | 55,4 | 57,4 |
| C/(A+B+C) | 0% | 1,5% | 2,9% | 5,7% | 8,3% | 10,7% | 0% | 3,5% | 6,7% | 9,8% | 12,6% |
| C/(A+C) | 0% | 9% | 17% | 29% | 38% | 44% | 0% | 17% | 29% | 38% | 44% |
| C/(A+B+C)) | 0% | 2% | 3% | 6% | 9% | 12% | 0% | 4% | 7% | 11% | 14% |
| B/(A+B) | 83,3% | 83,3% | 83,3% | 83,3% | 83,3% | 83,3% | 80% | 80% | 80% | 80% | 80% |
| Conformité aux critères du brevet | FAUX | FAUX | FAUX | FAUX | FAUX | FAUX | FAUX | VRAI | VRAI | FAUX | FAUX |
| Viscosité Brookfield à 120°C, mod 27 (mPa.s) | 35 | 65 | 125 | 280 | 4815 | 5875 | 3125 | 3500 | 3750 | 4250 | 2125 |
| Tack rolling ball / PSTC-6 / ASTM D3121 (mm) | 0 | 0 | 1093 | 903 | 1147 | 1330 | 970 | 670 | 800 | 1310 | >1500 |
| Force longitutinale pour allongement de 20% | 0 | 0 | 1 | 1,5 | 6 | 8,1 | 2,1 | 4,1 | 5,1 | 6,4 | 11,1 |
| Transparence | Ok | Ok | Léger | Blanc | Blanc | Blanc | Ok | Ok | Ok/léger | Blanc | Blanc |
| Tenue / manipulabilité | Mou | Mou | Mou | Mou | Ok | Cassant | Mou | Ok | Ok | Cassant | Cassant |
| Caractère Happant | Gras | Gras | Gras | Ok | Ok | Ok | Gras | Ok | Ok | Sec | Sec |
| Enductibilité | Facile | Facile | Facile | Facile | Facile | Facile | Facile | Facile | Facile | Facile | Facile |
| Conformité aux critères fonctionnels | | | | | | | | Bonnes caractéristiques fonctionnelles | Bonnes caractéristiques fonctionnelles | | |

## Tableau 2 (suite)

| Composition | Série HM-D2315' | | | | | Série HM-D2331' | | | | |
|---|---|---|---|---|---|---|---|---|---|---|
| | A | B | C | D | E | A | B | C | D | E |
| A (SIS) | 100 | 100 | 100 | 100 | 100 | 100 | 100 | 100 | 100 | 100 |
| B (Huile) | 350 | 350 | 350 | 350 | 350 | 300 | 300 | 300 | 300 | 300 |
| C (PE) | 0 | 20 | 40 | 60 | 80 | 0 | 20 | 40 | 60 | 80 |
| Carboxy Methyl Cellulose | 44,6 | 46,5 | 48,5 | 50,5 | 52,5 | 39,6 | 41,6 | 43,6 | 45,5 | 47,5 |
| | | | | | | | | | | |
| C/(A+B+C) | 0% | 3,9% | 7,4% | 10,7% | 13,7% | 0% | 4,3% | 8,3% | 11,9% | 15,17% |
| C/(A+C)) | 0% | 17% | 29% | 38% | 44% | 0% | 17% | 29% | 38% | 44% |
| C/(A+B+C)) | 0% | 4% | 8% | 12% | 15% | 0% | 5% | 9,1% | 13% | 17% |
| B/(A+B) | 77,8% | 77,8% | 77,8% | 77,8% | 77,8% | 75% | 75% | 75% | 75% | 75% |
| | | | | | | | | | | |
| Conformité aux critères du brevet | FAUX | VRAI | VRAI | FAUX | FAUX | FAUX | VRAI | FAUX | FAUX | FAUX |
| | | | | | | | | | | |
| Viscosité Brookfield à 120°C, mod 27 (mPa.s) | 2750 | 4375 | 6500 | 7000 | 1375 | 8250 | 13000 | 20000 | 4500 | 1750 |
| Tack rolling ball / PSTC-6 / ASTM D3121 | 895 | 738 | 1033 | 1255 | >1500 | 1070 | 790 | 1440 | >1500 | >1500 |
| Force longitudinale pour allongement de 20% | 2 | 3,5 | 4,8 | 9,8 | 11,7 | 3,1 | 4,8 | 6,5 | 14,3 | 16,8 |
| | | | | | | | | | | |
| Transparence | Ok | Ok | Léger | Blanc | Blanc | Ok | Ok/léger | Blanc | Blanc | Blanc |
| Tenue/ manipulabilité | Mou | Ok | Ok | Cassant | Cassant | Mou | Ok | Cassant | Cassant | Cassant |
| Caractère Happant | Gras | Ok | Ok | Sec | Sec | Gras | Ok | Sec | Sec | Sec |
| Enductibilité | Facile | Facile | Facile | Facile | facile | facile | difficile | Difficile | Difficile | Difficile |
| | | | | | | | | | | |
| Conformité aux critères fonctionnels | | Bonnes caractéristiques fonctionnelles | Bonnes caractéristiques fonctionnelles | | | | Bonnes caractéristiques fonctionnelles | | | |

EP 2 168 607 B1

**Revendications**

1. Interface chirurgicale pour plaie obtenue à partir d'une composition comprenant une matrice hydrophobe comportant A parties d'un élastomère tribloc, B parties d'un plastifiant, C parties d'une polyoléfine, et un hydrocolloïde dispersé dans une proportion comprise entre 0 et 30 % du poids total de la matrice hydrophobe, avec les relations suivantes :

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

la composition d'interface étant disposée en couche mince sur un support protecteur provisoire, des trous traversants répartis étant réalisés dans l'interface, l'interface étant ôtée de son support protecteur provisoire pour être utilisée et présentant une cohésion suffisante pour être manipulée et appliquée sur une plaie sans support ou armature.

2. Interface selon la revendication 1, **caractérisée en ce que** l'élastomère tribloc est un élastomère de type SIS, formé par copolymérisation de bloc de type polystyrène avec des blocs de type isoprène.

3. Interface selon l'une quelconque des revendications précédentes, **caractérisée en ce que** la polyoléfine est du polyéthylène.

4. Interface selon l'une quelconque des revendications précédentes, **caractérisée en ce que** le plastifiant est une huile de paraffine.

5. Interface selon les revendications 3 et 4, **caractérisée en ce qu'**il s'agit d'une interface autonome à partir d'une composition comprenant une matrice hydrophobe comportant A parties d'un élastomère tribloc, B parties d'huile de plastification, C parties de polyéthylène, et un hydrocolloïde dispersé dans une proportion comprise entre 5 et 15 % du poids total de la matrice hydrophobe, avec les relations suivantes :

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

la composition d'interface étant disposée en couche mince sur un support, des trous traversants répartis étant réalisés dans l'interface disposée sur le support, l'interface étant ôtée de son support pour être utilisée et présentant une cohésion suffisante pour être appliquée sur une plaie.

**6.** Interface selon l'une quelconque des revendications précédentes, **caractérisée en ce que** l'hydrocolloïde est de la carbométhylcellulose sodique (CMC).

**7.** Interface selon l'une quelconque des revendications précédentes, **caractérisée en ce que** l'interface est déposée par enduction sur un support.

**8.** Interface selon la revendication 6, **caractérisée en ce que** les trous traversants sont formés par perforation de l'enduction sur son support.

**9.** Interface selon la revendication 6, **caractérisée en ce que** les trous traversants sont formés par une enduction tramée.

**10.** Interface selon l'une quelconque des revendications précédentes, **caractérisée en ce qu'**elle est associée à une couche supplémentaire du côté opposée à la plaie.

**11.** Procédé de fabrication d'une interface chirurgicale pour plaie, **caractérisé en ce que** :

- on prépare une composition comprenant une matrice hydrophobe comportant A parties d'un élastomère tribloc, B parties d'un plastifiant, C parties d'une polyoléfine, et un hydrocolloïde dispersé dans une proportion comprise entre 0 et 30 % du poids total de la matrice hydrophobe, avec les relations suivantes :

$$A = 100$$

$$300 < B < 500$$

$$10 < C < 50$$

$$9\% < C/(A+C) < 33,5\%$$

$$1,5\% < C/(A + B + C) < 9\%$$

$$83\% > B/(A + B) > 75\%$$

- on enduit la composition d'interface en couche mince sur un support protecteur provisoire
- et **en ce qu'**on réalise des trous traversants répartis dans cette interface simultanément à l'enduction sur le support protecteur ou après celle-ci.

**12.** Utilisation d'une interface chirurgicale pour plaie selon l'une quelconque des revendications 1 à 10, **caractérisée en ce que** l'interface est ôtée de son support protecteur provisoire pour être utilisée et présentent une cohésion suffisante pour être manipulée et appliquée sur une plaie sans support ou armature.

**Claims**

1. Surgical interface for a wound, obtained from a composition including a hydrophobic matrix comprising A parts of a triblock elastomer, B parts of a plasticising oil, C parts of polyethylene, and a hydrocolloid dispersed in a proportion containing 0 to 30% of the total weight of the hydrophobic matrix, with the following relations:

$$A = 100$$

$$300 < B < 500$$

$$10 < C < 50$$

$$9\% < C/(A + C) < 33.5\%$$

$$1.5\% < C/(A + B + C) < 9\%$$

$$83\% > B/(A + B) > 75\%$$

wherein the interface composition is arranged in a thin layer on a temporary protective support, wherein distributed through-holes are formed in the said interface, and wherein the interface is removed from its temporary protective support in order to be used, and has sufficient cohesion for application to a wound without any support or reinforcement.

2. Interface according to claim 1, **characterised in that** the triblock elastomer is a SIS-type elastomer, formed by copolymerisation of polystyrene-type blocks with isoprene-type blocks.

3. Interface according to any one of the previous claims, **characterised in that** the polyeolefin is a polyethylene.

4. Interface according to any one of the previous claims, **characterised in that** the plasticizer is a paraffin oil.

5. Interface according to claims 3 and 4, **characterised in that** it is a self-supporting surgical interface based on a composition including a hydrophobic matrix comprising A parts of a triblock elastomer, B parts of a plasticising agent, C parts of polyethylene, and a hydrocolloid dispersed in a proportion containing 5 to 15% of the total weight of the hydrophobic matrix, with the following relations:

$$A = 100$$

$$300 < B < 500$$

$$10 < C < 50$$

$$9\% < C/(A + C) < 33.5\%$$

$$1.5\% < C/(A + B + C) < 9\%$$

$$83\% > B/(A + B) > 75\%$$

wherein the interface composition is arranged in a thin layer on a support, wherein distributed through-holes are formed in the interface arranged on the support, and wherein the interface is removed from its support in order to be used, and has sufficient cohesion for application to a wound.

6. Interface according to any one of the previous claims, **characterised in that** the hydrocolloid is sodium carboxymethyl cellulose (NaCMC).

7. Interface according to any one of the previous claims, **characterised in that** the interface is deposited by coating on a support.

8. Interface according to claim 6, **characterised in that** the through-holes are formed by perforation of the coating on its support.

9. Interface according to claim 6, **characterised in that** the through-holes are formed by a screened coating.

10. Interface according to any one of the previous claims, **characterised in that** it is associated with an additional layer on the side opposite the wound.

11. A manufacturing method of a surgical interface for wounds, **characterized in that** :

- A composition is prepared including a hydrophobic matrix comprising A parts of a triblock elastomer, B parts of a plasticising agent, C parts of polyolefin, and a hydrocolloid dispersed in the composition in a proportion containing 0 to 30% of the total weight of the hydrophobic matrix, with the following relations:

$$A = 100$$

$$300 < B < 500$$

$$10 < C < 50$$

$$9\% < C/(A + C) < 33.5\%$$

$$1.5\% < C/(A + B + C) < 9\%$$

$$83\% > B/(A + B) > 75\%$$

- coating the interface composition in a thin layer on a temporary protective support,
- and forming through-holes distributed **in that** interface either simultaneously with coating on the protective support, or after that step.

12. Use of a surgical interface for wounds according to anyone of claims 1 to 10, **characterised in that** interface is removed from its temporary protective support in order to be used, and has sufficient cohesion to be manipulated and applied to a wound without any support or reinforcement.

**Patentansprüche**

1. Chirurgische Schnittstelle für Wunde, erhalten durch eine Zusammensetzung, die eine hydrophobe Matrix mit A Anteilen eines Dreiblockelastomers, B Anteilen eines Weichmachers, C Anteilen eines Polyolefins, und einen Hydrokolloid umfasst, der in einem Verhältnis zwischen 0 und 30 % des Gesamtgewichts der hydrophoben Matrix dispergiert ist, mit den folgenden Beziehungen:

$$A = 100$$

$$300 < B < 500$$

$$10 < C < 50$$

$$9\% < C/(A + C) < 33,5\%$$

$$1,5\% < C/(A + B + C) < 9\%$$

$$83\% > B/(A + B) > 75\%$$

wobei die Zusammensetzung der Schnittstelle als dünne Lage auf einem vorläufigen Schutzträger angeordnet ist, und wobei verteilte durchgängige Löcher in der Schnittstelle ausgebildet sind, und wobei die Schnittstelle von ihrem Schutzträger zur Verwendung entfernt wird und eine ausreichende Kohäsion aufweist, um gehandhabt und auf eine Wunde ohne Stütze oder Verstärkung aufgebracht zu werden.

2. Schnittstelle nach Anspruch 1, **dadurch gekennzeichnet, dass** der Dreiblockelastomer ein Elastomer des SIS Typs ist, der durch Kopolymerisation von Blöcken des Polystyrol Typs mit Blöcken des Isopren Typs gebildet wird.

3. Schnittstelle nach einem der vorhergehenden Ansprüche, **dadurch gekennzeichnet, dass** das Polyolefin Polyethylen ist.

4. Schnittstelle nach einem der vorhergehenden Ansprüche, **dadurch gekennzeichnet, dass** der Weichmacher ein Paraffinöl ist.

5. Schnittstelle nach Anspruch 3 und 4, **dadurch gekennzeichnet, dass** es sich um eine autonome Schnittstelle handelt, ausgehend von einer Zusammensetzung, die eine hydrophobe Matrix mit A Anteilen eines Dreiblockelastomers, B Anteilen eines Plastifizierungsöls, C Anteilen Polyethylen, und einen Hydrokolloid umfasst, der in einem Verhältnis zwischen 5 und 15 % des Gesamtgewichts der hydrophoben Matrix dispergiert ist, mit den folgenden Beziehungen:

$$A = 100$$

$$300 < B < 500$$

$$10 < C < 50$$

$$9\% < C/(A + C) < 33,5\%$$

$$1,5\% < C/(A + B + C) < 9\%$$

$$83\% > B/(A + B) > 75\%$$

wobei die Zusammensetzung der Schnittstelle als dünne Lage auf einem Träger angeordnet ist, und wobei verteilte durchgängige Löcher in der auf dem Träger angeordneten Schnittstelle ausgebildet sind, und wobei die Schnittstelle von ihrem Träger zur Verwendung entfernt wird und eine ausreichende Kohäsion aufweist, um auf eine Wunde aufgebracht zu werden.

6. Schnittstelle nach einem der vorhergehenden Ansprüche, **dadurch gekennzeichnet, dass** der Hydrokolloid Natrium-Carboxymethylcellulose (CMC) ist.

7. Schnittstelle nach einem der vorhergehenden Ansprüche, **dadurch gekennzeichnet, dass** die Schnittstelle durch Beschichtung auf einem Träger aufgebracht ist.

8. Schnittstelle nach Anspruch 6, **dadurch gekennzeichnet, dass** die durchgehenden Löcher durch Perforation der Beschichtung auf seinem Träger gebildet werden.

9. Schnittstelle nach Anspruch 6, **dadurch gekennzeichnet, dass** die durchgehenden Löcher durch eine gerasterte Beschichtung gebildet werden.

10. Schnittstelle nach einem der vorhergehenden Ansprüche, **dadurch gekennzeichnet, dass** sie mit einer zusätzlichen Schicht verbunden ist, die sich auf der Seite befindet, die der Wunde gegenüberliegt.

11. Herstellungsverfahren einer chirurgische Schnittstelle für Wunde, **dadurch gekennzeichnet, dass**:

- Ansetzen einer Zusammensetzung, die eine hydrophobe Matrix mit A Anteilen eines Dreiblockelastomers, B Anteilen eines Weichmachers, C Anteilen eines Polyolefins, und einen Hydrokolloid umfasst, der in einem Verhältnis zwischen 0 und 30 % des Gesamtgewichts der hydrophoben Matrix dispergiert ist, mit den folgenden Beziehungen:

$$A = 100$$

$$300 < B < 500$$

$$10 < C < 50$$

$$9\% < C/(A + C) < 33,5\%$$

$$1,5\% < C/(A + B + C) < 9\%$$

$$83\% > B/(A + B) > 75\%$$

- Auftragen der Zusammensetzung der Schnittstelle als dünne Lage auf einem vorläufigen Schutzträger als Schicht, und
- und Realisation von auf dieser Schnittstelle verteilte durchgängige Löcher in der Schnittstelle gleichzeitig mit oder nach der Beschichtung auf dem Schutzträger.

12. Verwendung einer chirurgischen Schnittstelle für Wunde nach einem der Ansprüche von 1 bis 10, **dadurch ge-**

**kennzeichnet, dass** die Schnittstelle von ihrem Schutzträger zur Verwendung entfernt wird und eine ausreichende Kohäsion aufweist, um gehandhabt und auf eine Wunde ohne Stütze oder Verstärkung aufgebracht zu werden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1143895 A
- WO 9927014 A
- US 20050123590 A
- WO 011352 A